# EUROPEAN PATENT SPECIFICATION

(11) **EP 4 285 978 B1**
(45) Date of publication and mention of the grant of the patent: **16.09.2026**
(21) Application number: 22756337.6
(22) Date of filing: 22.02.2022
(51) Int. Cl.: A61M 25/00

(54) **CATHETER**
KATHETER
CATHÉTER

(30) Priority: 22.02.2021 JP 2021026036
(43) Date of publication of application: 06.12.2023
(73) Proprietor: TERUMO Kabushiki Kaisha, Tokyo 151-0072 (JP)
(72) Inventor: KITAMURA, Kodai, Ashigarakami-gun, Kanagawa 259-0151 (JP); ITO, Takashi, Fujinomiya-shi, Shizuoka 418-0015 (JP); HOSHI, Mitsuki, Fujinomiya-shi, Shizuoka 418-0015 (JP)
(74) Representative: Casalonga
(86) International application number: PCT/JP2022/007144
(87) International publication number: WO 2022/177020

(56) References cited:
- EP-A1- 2 695 635
- EP-B1- 3 082 930
- WO-A1-2019/004100
- JP-A- 2014 144 163
- JP-A- 2014 155 606
- US-A1- 2017 095 640

## Description

### Technical Field

The present invention relates to a catheter used in a lumen such as a blood vessel. In particular, the present invention relates to a catheter according to the preamble of independent claim 1 or independent claim 3, such as they are e.g. known from US 2017/095640 A1.

### Background Art

In recent years, intraluminal treatment of a blood vessel or the like using a catheter has been actively performed because surgical invasion is very low. It is desired that the catheter is thin while maintaining strength in a manner that the catheter can be inserted into a thin lumen and a wide passage can be secured inside.

As a method of making a catheter have high strength and thin thickness, a flat wire, which is a strip-shaped wire rod made of a flat plate, is braided into a tubular shape and embedded in the catheter as a reinforcing body (see, for example, Patent Literature 1).

### Citation List

### Patent Literature

Patent Literature 1: JP 2014-144163 A

### Summary of Invention

### Technical Problem

However, since the flat plate has high strength, when the flat wire is braided, the cut end portion of the flat plate is likely to be separated. As a result, there is a possibility that the catheter cannot be easily cut, or when the reinforcing body is embedded in the tubular resin material, the separated reinforcing body cannot come into close contact with the resin material, and the strength of the catheter decreases. For this reason, in general, in a catheter using a flat wire as a reinforcing body, post-processing such as welding wire rods at end portions of the reinforcing body is required.

The present invention has been made to solve the above-described problems, and an object of the present invention is to provide a catheter capable of suppressing separation of an end portion of a reinforcing body while applying a flat wire effective for making the catheter thin and have high strength to the reinforcing body.

### Solution to Problem

The above problem is solved by a catheter according to independent claim 1 or a catheter according to independent claim 3. The dependent claims relate to advantageous embodiments.

### Advantageous Effects of Invention

In a catheter configured as described above, it is possible to suppress the end portion of the reinforcing body from being separated by the round wire group which is applied to the reinforcing body together with the flat wire and intersects with the flat wire while applying the flat wire effective for making the catheter thin and high strength to the reinforcing body.

The cross-sectional area ratio may be larger than 1. As a result, the catheter can enhance the effect that the end portion of the reinforcing body is less likely to be separated due to the round wire group.

The yield point of the material of the round wire is lower than the yield point of the material of the flat wire. As a result, the round wire is more easily plastically deformed than the flat wire, and the braided shape is easily maintained. Therefore, it is possible to effectively suppress the end portion of the reinforcing body from being separated by holding the flat wire which is made to be separated because it is difficult to plastically deform by the round wire group.

The number of the round wires may be larger than the number of the flat wires. As a result, it is possible to effectively suppress the end portion of the reinforcing body from being separated by holding the flat wire which is made to be separated by the round wire.

The intersecting wire rods may not be joined to each other. As a result, in the catheter, even if the intersecting wire rods are not joined to each other, it is possible to suppress separation of the reinforcing body. Therefore, it is possible to facilitate processing of the thin catheter with high strength.

The flat wire may be a wire rod having a rectangular cross section. As a result, the cross-sectional area of the flat wire increases, and the flat wire can be thinned to obtain the thin catheter with high strength.

In the catheter of still another aspect configured as described above, it is possible to suppress the end portion of the reinforcing body from being separated by the round wire which is applied to the reinforcing body together with the flat wire and intersects with the flat wire while applying the flat wire effective for making the catheter thin and high strength to the reinforcing body.

### Brief Description of Drawings

Fig. 1 is a plan view illustrating a catheter according to an embodiment.
Fig. 2 is a longitudinal sectional view along a central axis, illustrating the catheter according to the embodiment.
Fig. 3 is a lateral longitudinal sectional view orthogonal to a central axis, illustrating the catheter according to the embodiment.
Fig. 4 is a plan view illustrating an outer layer of the catheter according to the embodiment in a transparent manner.
Figs. 5(A) and 5(B) are cross-sectional views illustrating a wire rod of a reinforcing body, in which Fig. 5(A) illustrates a round wire and Fig. 5(B) illustrates a flat wire.
Fig. 6 is a plan view illustrating, as a reference example, a state in which a wire rod is separated at an end portion of a cut reinforcing body in a manufacturing process.

### Description of Embodiments

Hereinafter, embodiments of the invention will be described with reference to the accompanying drawings. Note that the dimensions of the drawings may be exaggerated and different from actual dimensions for convenience of description. In the present specification and the drawings, components having substantially the same functional configuration are denoted by the same reference numerals, and the overlapping description will be omitted. In the present specification, a side to be inserted into a body lumen of a catheter 1 is referred to as a "distal end side", and a side to be operated is referred to as a "proximal end side".

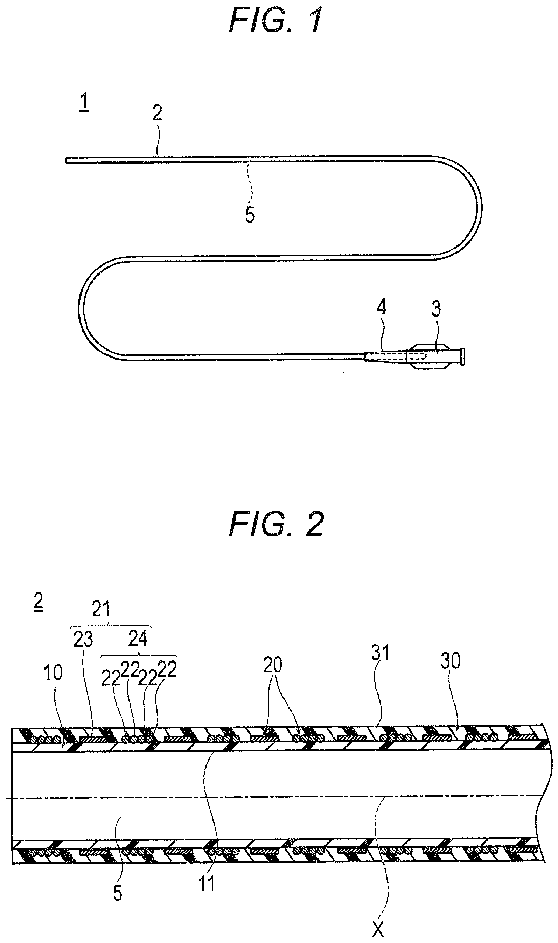

The catheter 1 according to the present embodiment may be introduced into a blood vessel from a radial artery of an arm and inserted into an artery of a lower limb, and is used for performing treatment, diagnosis, or the like. The artery of the lower limb is an artery near the aortoiliac artery bifurcation and on the more peripheral side. As illustrated in Fig. 1, the catheter 1 includes an elongated shaft 2, a hub 3 connected to a proximal end of the shaft 2, and a kink protector 4 provided at a connection portion between the shaft 2 and the hub 3.

As illustrated in Figs. 1 to 4, the shaft 2 is a tubular member having flexibility, has a central axis X extending from a proximal end to a distal end, and has a lumen 5 formed inside from the proximal end to the distal end. A guide wire is inserted through the lumen 5 when the catheter 1 is inserted into a blood vessel. In addition, the lumen 5 can also be used as a passage for medical instruments such as other catheters 1, medicinal solutions, embolic substances, contrast media, or the like.

The effective length of the shaft 2 is not particularly limited, and is appropriately set according to the application of the catheter 1. In a case where the catheter 1 is introduced into a blood vessel from a radial artery of an arm and inserted into an artery of a lower limb, and is used for performing treatment, diagnosis, or the like, the effective length of the shaft 2 is not particularly limited, but is preferably 1500 mm to 2600 mm, more preferably 1800 mm to 2300 mm, and still more preferably 2100 mm to 2300 mm. As a result, the catheter 1 can reach the artery of the lower limb from the artery of the arm. Note that the effective length of the shaft 2 is a length of a portion that can be inserted into a blood vessel, a sheath, or the like. In the present embodiment, the effective length is a length along the central axis X from the most distal end of the kink protector 4 to the most distal end of the shaft 2. The effective length of the shaft 2 is preferably 650 mm or more in a case where the catheter 1 is introduced into the blood vessel from the femoral artery, and is preferably 300 mm or more in a case where the catheter 1 is introduced into the blood vessel from the distal portion of the dorsalis pedis artery or the posterior tibial artery.

The shaft 2 includes a plurality of layers, and includes an inner layer 10 forming an inner surface 11 of the lumen 5, a reinforcing body 20 disposed on the outer side of the inner layer 10, and an outer layer 30 formed on the outer side of the inner layer 10 and the reinforcing body 20.

The lumen 5 is formed inside the inner layer 10. As a constituent material of the inner layer 10, a thermoplastic resin, a thermosetting resin, or the like can be applied, and a fluorine-based resin such as polytetrafluoroethylene (PTFE), a low-friction material such as high-density polyethylene (HDPE), or the like is preferable.

The inner diameter of the inner layer 10 is not particularly limited, but is preferably 0.4 mm to 1.2 mm, more preferably 0.45 mm to 0.7 mm, and still more preferably 0.5 mm to 0.6 mm.

The reinforcing body 20 is formed by braiding a plurality of wires 21 in a tubular shape to have a gap on the outer periphery of the inner layer 10. The wires 21 includes a plurality of round wire 22 having a circular cross section and a plurality of flat wires 23 having a rectangular cross section. The cross section of the wire 21 is a cross section orthogonal to the direction in which the wire 21 extends. The cross section of the flat wire 23 may be a rectangle, an ellipse, or an oval, but in a case where shapes are compared with the same thickness and width, a rectangle having the largest cross-sectional area is preferable.

The round wires 22 forms one round wire group 24 with a plurality of strands. Each round wire group 24 includes one or a plurality of round wires 22 arranged adjacent to each other in the circumferential direction of the shaft 2. The round wire group 24 extends to draw a spiral in the same direction around the outer periphery of the inner layer 10, and is disposed at equal intervals in the circumferential direction of the shaft 2. The plurality of round wires 22 constituting each round wire group 24 are disposed side by side without a gap in the circumferential direction of the shaft 2. Note that the plurality of round wires 22 constituting each round wire group 24 may be disposed with some gap in the circumferential direction of the shaft 2. A number L1 of the round wire groups 24 is equal to the number of the flat wires 23, but may not be equal to it. The number L1 of the round wire groups 24 is not particularly limited in a case where one of the round wire groups 24 is 1 line, but is, for example, 1 line to 16 lines, and is 4 lines in the present embodiment. A number N1 of the round wires 22 included in each round wire group 24 is not particularly limited, but is, for example, 1 wire to 8 wires, and is 4 wires in the present embodiment. In a case where the plurality of round wires 22 is included in one round wire group 24, the plurality of round wires 22 having a small outer diameter can be used instead of one round wire 22 having a large outer diameter, in a manner that it is possible to suppress an increase in the wall thickness and the outer diameter of the shaft 2.

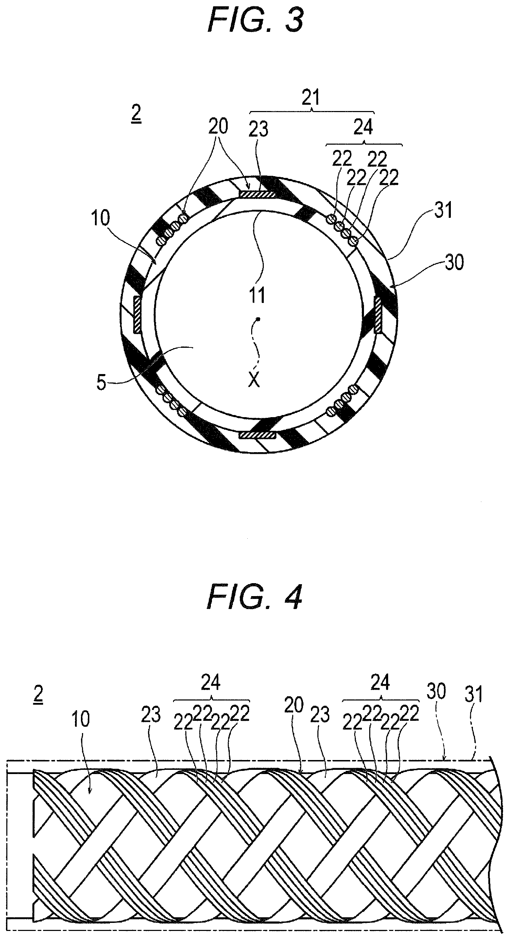

The flat wires 23 extend in the same direction in a manner that 1 wire of a strand draws a spiral around the outer periphery of the inner layer 10, and are disposed at equal intervals in the circumferential direction of the shaft 2. The direction in which the plurality of flat wires 23 extend is opposite to the direction in which the round wire group 24 extends in the circumferential direction of the shaft 2 to intersect with the round wire group 24. The flat wire 23 and the round wire group 24 are braided in a manner that the arrangement overlapping in the radial direction of the shaft 2 is interchanged alternately or with a pattern. As illustrated in Fig. 3, a number L2 of the flat wires 23 is not particularly limited in a case where 1 wire of a flat wire is defined as 1 line, but is, for example, 1 line to 16 lines. The number L2 of the flat wires 23 matches the number L1 of the round wire groups 24, but does not necessarily need to match. Each flat wire 23 is disposed in a manner that the direction of the short side of the rectangular cross section substantially matches the radial direction of the shaft 2.

As a constituent material of the round wire 22 and the flat wire 23, a metal wire such as stainless steel, platinum (Pt), tungsten (W), or the like, a resin fiber, a carbon fiber, a glass fiber, or the like can be applied, or a plurality of these wires 21 may be used in combination.

The constituent materials of the round wire 22 and the flat wire 23 may be the same material, but are different in embodiments forming part of the claimed invention. The yield point of the material of the round wire 22 is lower than the yield point of the material of the flat wire 23. The round wire 22 is deformed beyond the yield point in the braided state. That is, the round wire 22 is plastically deformed when braided, and constitutes the reinforcing body 20 in a state where the restoring force to return to the original shape is smaller than that of the flat wire 23. The flat wire 23 constitutes the reinforcing body 20 in a state where the internal stress remains larger than that of the round wire 22 in the braided state and the restoring force for returning to the original shape is larger than that of the round wire 22.

As an example, the constituent materials of the round wire 22 and the flat wire 23 are different stainless steel, and the yield point of the material of the round wire 22 is lower than the yield point of the material of the flat wire 23. A constituent material of the round wire 22 is, for example, SUS316, and a constituent material of the flat wire 23 is, for example, SUS304-WPB.

In a case where the total (total cross-sectional area) of the cross-sectional areas of all the round wires 22 constituting the reinforcing body 20 is defined as A, and the total (total cross-sectional area) of the cross-sectional areas of all the flat wires 23 constituting the reinforcing body 20 is defined as B, A/B is preferably larger than 0.5, more preferably 0.74 or more, more preferably larger than 1, still more preferably 1.4 or more, and still more preferably 2.0 or more. A/B is preferably 2.1 or less, more preferably less than 2.1, and still more preferably 2.09 or less. As a result, it is possible to sufficiently secure the total cross-sectional area of the round wires 22 and improve the effect of maintaining the shape of the cut end portion of the reinforcing body 20 by the plastically deformed round wires 22. Note that the cross-sectional area of the wire 21 is an area in a cross section orthogonal to the extending direction of the wire 21.

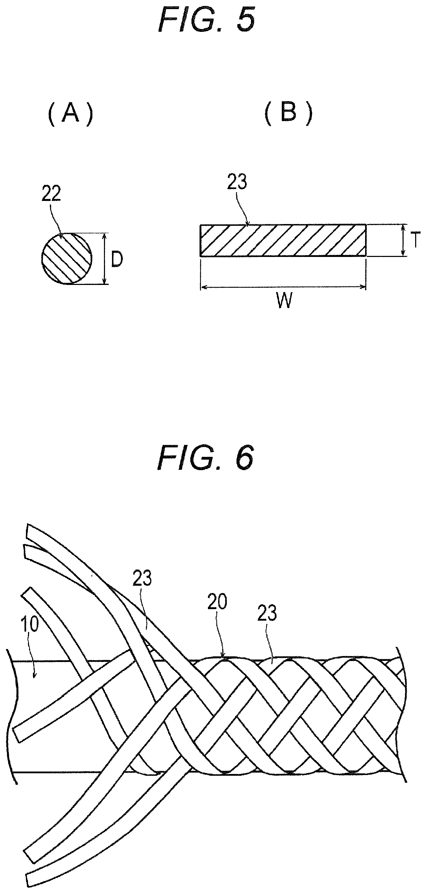

In addition, as illustrated in Fig. 5, in a case where the diameter of the round wire 22 is defined as D and the thickness of the flat wire 23 (the length of the short side of the rectangular cross section) is defined as T, D/T is preferably larger than 1.5, more preferably 2.0 or more, and still more preferably 4.0 or more. As a result, it is possible to sufficiently secure the diameter of the round wire 22 and improve the effect of maintaining the shape of the cut end portion of the reinforcing body 20 by the plastically deformed round wire 22. Note that a diameter D of the round wire 22 is a diameter in a cross section orthogonal to the extending direction of the round wire 22. A thickness T of the flat wire 23 is the length of the short side of the rectangular cross section in the cross section orthogonal to the extending direction of the flat wire 23. A width W of the flat wire 23 is the length of the long side of the rectangular cross section in the cross section orthogonal to the extending direction of the flat wire 23.

The diameter D of the round wire 22 is not particularly limited, but is preferably 0.020 mm to 0.080 mm, more preferably 0.025 mm to 0.060 mm, and still more preferably 0.030 mm to 0.040 mm.

The thickness T of the flat wire 23 is not particularly limited, but is preferably 0.010 mm to 0.040 mm, more preferably 0.010 mm to 0.030 mm, and still more preferably 0.015 mm to 0.020 mm. The width W of the flat wire 23 is not particularly limited, but is preferably 0.045 mm to 0.250 mm, more preferably 0.045 mm to 0.200 mm, and still more preferably 0.045 mm to 0.140 mm.

As illustrated in Figs. 2 to 4, the outer layer 30 is a tubular member that covers the outer periphery of the inner layer 10 and the reinforcing body 20. The outer layer 30 forms an outer surface 31 which is a radially outer surface of the shaft 2.

The outer diameter of the outer layer 30 is not particularly limited, but is preferably 0.7 mm to 1.3 mm, more preferably 0.8 mm to 1.2 mm, and still more preferably 0.86 mm to 1.1 mm.

As a constituent material of the outer layer 30, for example, a polymer material such as polyolefin (for example, polyethylene, polypropylene, polybutene, an ethylene-propylene copolymer, an ethylene-vinyl acetate copolymer, an ionomer, or a mixture of two or more kinds of these), polyvinyl chloride, polyamide, a polyester elastomer, a polyamide elastomer, polyurethane, a polyurethane elastomer, polyimide, or a fluororesin, a thermoplastic resin such as a mixture of these, or a thermosetting resin such as an epoxy resin can be applied. The radiopaque substance may be mixed with the outer layer 30.

As illustrated in Fig. 1, in the hub 3, a proximal end portion of the shaft 2 is liquid-tightly fixed by an adhesive, heat fusion, a stopper (not illustrated), or the like. The hub 3 functions as a guide wire or an insertion port of a medical instrument into the lumen 5, an injection port of a medicinal solution, an embolic substance, a contrast medium, or the like into the lumen 5, and also functions as a grip portion when the catheter 1 is operated. The constituent material of the hub 3 is not particularly limited, but for example, thermoplastic resins such as polycarbonate, polyamide, polysulfone, polyarylate, and a methacrylate-butylene-styrene copolymer can be suitably used.

The kink protector 4 is made of an elastic material provided to surround the periphery of the shaft 2, and suppresses kink of the shaft 2 at a connection portion between the shaft 2 and the hub 3. As a constituent material of the kink protector 4, for example, natural rubber, silicone resin, polyamide elastomer, polyester elastomer, or the like can be suitably used.

Next, a method for manufacturing the catheter 1 of the present invention will be described.

First, a long core wire having an outer diameter equal to the inner diameter of the inner layer 10 is prepared. Next, the inner layer 10 is formed on the core wire. The inner layer 10 may be formed by extrusion or by dip forming. Alternatively, the core wire may be inserted into the lumen 5 of the inner layer 10 which is a tubular body.

Thereafter, as shown in Fig. 4, the reinforcing body 20 is formed to cover at least a part of the inner layer 10. The reinforcing body 20 is formed by continuously winding a plurality of round wires 22 and flat wires 23 on the inner layer 10 using a braiding machine (braider). The flat wire 23 can impart high strength to the shaft 2 while maintaining the formed shaft 2 thin. The flat wire 23 constitutes the reinforcing body 20 in a state where the internal stress remains larger than that of the round wire 22 in the braided state and the restoring force for returning to the original shape is larger than that of the round wire 22. The round wire 22 is deformed beyond the yield point in the braided state. That is, the round wire 22 is plastically deformed when braided, and constitutes the reinforcing body 20 in a stable state where the restoring force to return to the original shape is smaller than that of the flat wire 23.

Next, the end portion of the reinforcing body 20 is cut. At this time, the cut end portion of the flat wire 23 having high strength and a high yield point is likely to be spread and separated by its own restoring force as in the reference example of Fig. 6. Here, the separation means that the tubular wound reinforcing wire (for example, the wires 21) spreads to five times or more the outer diameter in a natural state. However, in the present embodiment, since the flat wire 23 intersects with the round wires 22 which is plastically deformed and has small and stable internal stress, the shape of the end portion of the cut flat wire 23 is held by the round wires 22. Therefore, the reinforcing body 20 can suppress the separation of the wires 21 (the round wires 22 and the flat wire 23) at the cut end portion.

Next, the reinforcing body 20 is pressed from the outer peripheral surface side, and the inner peripheral surface side of the reinforcing body 20 is embedded in the inner layer 10. Thereafter, as shown in Figs. 2 to 3, the outer layer 30 is formed on the outer side of the inner layer 10 and the reinforcing body 20. A method for forming the outer layer 30 is not particularly limited. For example, the outer layer 30 may be formed by extrusion molding or dip molding. Alternatively, the outer layer 30 may be formed by disposing a tubular body as a material of the outer layer 30 on the outer side of the inner layer 10 and the reinforcing body 20, and then covering the tubular body with a heat shrinkable tube and heating the tubular body. While being softened or melted by heating, the tubular body is joined in close contact with the outer side of the inner layer 10 and the reinforcing body 20 by the contraction force of the heat shrinkable tube. After this, the heat shrinkable tube is removed.

After forming the outer layer 30, the core wire is pulled out from the lumen 5 of the inner layer 10. After this, the hub 3 and the kink protector 4 are attached to the shaft 2, and other members (for example, the distal end chip) are attached as necessary to complete the catheter 1. Alternatively, the distal end chip may be attached from the beginning and molded.

As described above, the catheter 1 according to the present invention is the catheter 1 including the shaft 2 having the lumen 5 communicating from the distal end to the proximal end, in which the shaft 2 has the reinforcing body 20 including the wire 21 disposed at least partially between the inner surface 11 of the shaft 2 forming the lumen 5 and the outer surface 31 of the shaft 2 and braided in a tubular shape, the reinforcing body 20 has the round wire group 24 including the plurality of round wires 22 which are the wires 21 having a circular cross section and the plurality of flat wires 23 which are the wires 21 having a rectangular cross section and intersecting the round wire group 24, and the cross-sectional area ratio A/B of the total cross-sectional area A of the plurality of round wire groups 24 to the total cross-sectional area B of the plurality of flat wires 23 is larger than 0.5.

Alternatively, the catheter 1 according to the present invention is a catheter 1 catheter including a shaft 2 having a lumen 5 communicating from a distal end to a proximal end, in which the shaft 2 includes a reinforcing body 20 including a wire 21 disposed on at least a part between an inner surface 11 of the shaft 2 forming the lumen 5 and an outer surface 31 of the shaft 2 and braided in a tubular shape, the reinforcing body 20 includes a round wire group 24 including a plurality of round wires 22 each of which is a wire 21 having a circular cross section, and a plurality of flat wires 23 each of which is a wire 21 having a rectangular cross section and intersecting the round wire group 24, and a ratio D/T of a diameter D of the round wire 22 to a thickness T of the flat wire 23 is larger than 1.5.

In the catheter 1 configured as described above, it is possible to suppress the end portion of the reinforcing body 20 from being separated by the round wire group 24 which is applied to the reinforcing body 20 together with the flat wire 23 and intersects with the flat wire 23 while applying the flat wire 23 effective for making the catheter 1 thin and high strength to the reinforcing body 20. Therefore, it is possible to facilitate processing of the thin catheter 1 with high strength. Here, the separation means that the maximum outer diameter of the end portion of the reinforcing body 20 when the reinforcement wire (wire rod of the reinforcing body 20) is cut is five times or more the outer diameter of the intermediate portion of the reinforcing body 20 that is not separated.

In addition, the cross-sectional area ratio A/B may be larger than 1. As a result, it is possible to enhance the effect that the end portion of the reinforcing body 20 is less likely to be separated due to the round wire group 24.

In addition, the yield point of the material of the round wire 22 is lower than the yield point of the material of the flat wire 23. As a result, the round wire22 is more easily plastically deformed than the flat wire 23, and the braided shape is easily maintained. Therefore, it is possible to effectively suppress the end portion of the reinforcing body 20 from being separated by holding the flat wire 23 which is made to be separated because it is difficult to plastically deform by the round wire group 24.

In addition, the number of round wires 22 is larger than the number of flat wires 23. As a result, it is possible to effectively suppress the end portion of the reinforcing body 20 from being separated by holding the flat wire 23 which is made to be separated by the round wire 22.

In addition, the intersecting wires 21 are not joined to each other by welding, bonding, or the like. In the catheter 1, even if the intersecting wires 21 are not joined to each other, it is possible to suppress separation of the reinforcing body 20. Therefore, it is possible to facilitate processing of the thin catheter 1 with high strength.

In addition, the effective length of the shaft 2 is 2100 mm or more. As a result, the catheter 1 can easily reach the artery of the lower limb from the artery of the arm.

In addition, the flat wire 23 is a wire rod having a rectangular cross section. As a result, the cross-sectional area of the flat wire 23 increases, and the flat wire 23 can be thinned to obtain the thin catheter 1 with high strength.

As a modification, strands of the round wire 22 and the flat wire 23 may be braided. In the braided catheter configured as described above, it is possible to suppress the end portion of the reinforcing body 20 from being separated by the round wire 22 which is applied to the reinforcing body 20 together with the flat wire 23 and intersects with the flat wire 23 while applying the flat wire 23 effective for making the catheter thin and high strength to the reinforcing body 20.

### Examples

Hereinafter, examples and comparative examples of the present invention will be described. Note that the present invention is not limited to these examples.

The shaft 2 of Examples 1 to 5 and Comparative Example 1 shown in Table 1 has been produced, and whether or not the cut end portion of the reinforcing body 20 becomes separated has been observed in the process of producing the shaft 2. In all of Examples 1 to 5 and Comparative Example 1, the material of the round wire 22 is SUS316 and the material of the flat wire 23 is SUS304-WPB.

Note that, in Example 1, the disposition of the round wires 22 has been braided by setting the braiding machine in a manner that the number L1 of the round wire groups 24 is 8 lines, and the number N1 of the round wires 22 constituting each round wire group 24 is 2 wires, but after the braiding, the two adjacent round wire groups 24 have been combined, and substantially, the number L1 of the round wire groups 24 has been 4 lines, and the number N1 of the strands of the round wires 22 constituting each round wire group 24 has been 4 wires.

As a result, in Examples 1 to 5 in which the cross-sectional area ratio A/B is larger than 0.5 and smaller than 2.1, the result that the cut end portions of the reinforcing body 20 are not separated (or hardly separated) has been obtained, as compared with Comparative Example 1 in which the cross-sectional area ratio A/B is smaller than 0.5. In addition, in Examples 1 to 5 in which a thickness ratio D/T is larger than 1.5, the result that the cut end portions of the reinforcing body 20 are not separated (or hardly separated) has been obtained, as compared with Comparative Example 1 in which the thickness ratio D/T is smaller than 1.5.

**[Table 1]**

| | Round wire | | | | Flat wire | | | | | Cross-sectional area ratio A/B | Thickness ratio D/T | Result |
|---|---|---|---|---|---|---|---|---|---|---|---|---|
| | wire composition N1 × L1 | Wire diameter D [mm] | Number of wires | Total cross-sectional area A [mm²] | wire composition L2 | Thickness T [mm] | width W [mm] | Number of wires | Total cross-sectional area B [mm²] | | | |
| Example 1 | 2 wires × 8 lines | 0.04 | 16 | 0.020 | 4 lines | 0.02 | 0.17 | 4 | 0.014 | 1.48 | 2.00 | Not separated |
| Example 2 | 2 wires × 4 lines | 0.04 | 8 | 0.010 | 4 lines | 0.02 | 0.17 | 4 | 0.014 | 0.74 | 2.00 | Not separated |
| Example 3 | 1 wire × 4 lines | 0.08 | 4 | 0.020 | 4 lines | 0.02 | 0.17 | 4 | 0.014 | 1.48 | 4.00 | Not separated |
| Comparative Example 1 | 2 wires × 4 lines | 0.03 | 8 | 0.006 | 4 lines | 0.02 | 0.17 | 4 | 0.014 | 0.42 | 1.50 | Becomes separated |
| Example 4 | 2 wires × 4 lines | 0.03 | 8 | 0.006 | 4 lines | 0.015 | 0.045 | 4 | 0.003 | 2.09 | 2.00 | Not separated |
| Example 5 | 2 wires × 8 lines | 0.03 | 16 | 0.011 | 8 lines | 0.01 | 0.08 | 8 | 0.006 | 1.76 | 3.00 | Not separated |

The invention is not limited to the above embodiments, and various modifications may be made within the technical idea of the invention by those skilled in the art. The catheter 1 may be inserted from a blood vessel other than the artery of the arm. In addition, the catheter 1 may be used for treatment or diagnosis of a blood vessel other than an artery of a lower limb. In addition, the catheter 1 may be inserted into a bile duct, a trachea, an esophagus, a urethra, or other body lumen or body cavity to be used for treatment, diagnosis, or the like.

### Reference Signs List

- 1: Catheter
- 2: Shaft
- 5: Lumen
- 10: Inner layer
- 11: Inner surface
- 20: Reinforcing body
- 21: Wire rod
- 22: Round wire
- 23: Flat wire
- 24: Round wire group
- 30: Outer layer
- 31: Outer surface
- A: Total cross-sectional area of round wire
- B: Total cross-sectional area of flat wire
- D: Diameter of round wire
- T: Thickness of flat wire
- W: Width of flat wire

## Claims

1. A catheter (1) comprising a shaft (2) having a lumen (5) communicating from a distal end to a proximal end, wherein
the shaft (2) includes a reinforcing member including a wire rod (21) disposed on at least a part between an inner surface (11) of the shaft (2) forming the lumen (5) and an outer surface (31) of the shaft (2) and braided in a tubular shape,
the reinforcing body (20) includes a round wire group (24) including a plurality of round wires (22) each of which is a wire rod (21) having a circular cross section, and a plurality of flat wires (23) intersecting the round wire group (24), wherein the round and flat wires are part of the braid of the wire rod, and
a cross-sectional area ratio of a total cross-sectional area of the plurality of round wires (22) of the round wire group (24) to a total cross-sectional area (B) of the plurality of flat wires (23) is larger than 0.5,
**characterized in that**
a yield point of a material of the round wires (22) is lower than a yield point of a material of the flat wires (23), and the round wires (22) are deformed beyond the yield point in the braided state.

2. The catheter (1) according to claim 1, wherein the cross-sectional area ratio is larger than 1.

3. A catheter (1) comprising a shaft (2) having a lumen (5) communicating from a distal end to a proximal end, wherein
the shaft (2) includes a reinforcing member including a wire rod (21) disposed on at least a part between an inner surface (11) of the shaft (2) forming the lumen (5) and an outer surface (31) of the shaft (2) and braided in a tubular shape,
the reinforcing body (20) includes a round wire group (24) including a plurality of round wires (22) each of which is a wire rod (21) having a circular cross section, and a plurality of flat wires (23) intersecting the round wire group (24), wherein the round and flat wires are part of the braid of the wire rod, and wherein
a ratio of a diameter (D) of the round wires wire(22) to a thickness (T) of the flat wires (23) is larger than 1.5,
**characterized in that** a yield point of a material of the round wires (22) is lower than a yield point of a material of the flat wires (23), and the round wires (22) are deformed beyond the yield point in the braided state.

4. The catheter (1) according to any one of claims 1 to 3, wherein a number of the round wires (22) is larger than a number of the flat wires (23).

5. The catheter (1) according to any one of claims 1 to 4, wherein the intersecting wire rods (21) are not joined to each other.

6. The catheter (1) according to any one of claims 1 to 4, wherein the flat wire (23) is a wire rod (21) having a rectangular cross section.

## Patentansprüche

1. Katheter (1), der einen Schaft (2) umfasst, der ein Lumen (5) aufweist, das von einem distalen Ende bis zu einem proximalen Ende verbindet, wobei
der Schaft (2) ein Verstärkungselement einschließt, das einen Walzdraht (21) einschließt, der an zumindest einem Teil zwischen einer Innenfläche (11) des Schafts (2), die das Lumen (5) bildet, und einer Außenfläche (31) des Schafts (2) angeordnet und in einer röhrenförmigen Gestalt geflochten ist,
der Verstärkungskörper (20) eine Runddrahtgruppe (24), die eine Vielzahl von Runddrähten (22) einschließt, von denen jeder ein Walzdraht (21) ist, der einen kreisförmigen Querschnitt aufweist, und eine Vielzahl von Flachdrähten (23), welche die Runddrahtgruppe (24) überschneiden, einschließt, wobei die Rund- und die Flachdrähte Teil des Geflechts des Walzdrahtes sind, und
ein Querschnittsflächenverhältnis einer Gesamtquerschnittsfläche der Vielzahl von Runddrähten (22) der Runddrahtgruppe (24) zu einer Gesamtquerschnittsfläche (B) der Vielzahl von Flachdrähten (23) größer als 0,5 ist,
**dadurch gekennzeichnet, dass**
eine Streckgrenze eines Materials der Runddrähte (22) niedriger ist als eine Streckgrenze eines Materials der Flachdrähte (23) und die Runddrähte (22) in dem geflochtenen Zustand über die Streckgrenze hinaus verformt sind.

2. Katheter (1) nach Anspruch 1, wobei das Querschnittsflächenverhältnis größer als 1 ist.

3. Katheter (1), der einen Schaft (2) umfasst, der ein Lumen (5) aufweist, das von einem distalen Ende bis zu einem proximalen Ende verbindet, wobei
der Schaft (2) ein Verstärkungselement einschließt, das einen Walzdraht (21) einschließt, der an zumindest einem Teil zwischen einer Innenfläche (11) des Schafts (2), die das Lumen (5) bildet, und einer Außenfläche (31) des Schafts (2) angeordnet und in einer röhrenförmigen Gestalt geflochten ist,
der Verstärkungskörper (20) eine Runddrahtgruppe (24), die eine Vielzahl von Runddrähten (22) einschließt, von denen jeder ein Walzdraht (21) ist, der einen kreisförmigen Querschnitt aufweist, und eine Vielzahl von Flachdrähten (23), welche die Runddrahtgruppe (24) überschneiden, einschließt, wobei die Rund- und die Flachdrähte Teil des Geflechts des Walzdrahtes sind, und wobei
ein Verhältnis eines Durchmessers (D) der Runddrähte (22) zu einer Dicke (T) der Flachdrähte (23) größer als 1,5 ist,
**dadurch gekennzeichnet, dass** eine Streckgrenze eines Materials der Runddrähte (22) niedriger ist als eine Streckgrenze eines Materials der Flachdrähte (23) und die Runddrähte (22) in dem geflochtenen Zustand über die Streckgrenze hinaus verformt sind.

4. Katheter (1) nach einem der Ansprüche 1 bis 3, wobei eine Anzahl der Runddrähte (22) größer ist als eine Anzahl der Flachdrähte (23).

5. Katheter (1) nach einem der Ansprüche 1 bis 4, wobei die überschneidenden Walzdrähte (21) nicht miteinander verbunden sind.

6. Katheter (1) nach einem der Ansprüche 1 bis 4, wobei der Flachdraht (23) ein Walzdraht (21) ist, der einen rechteckigen Querschnitt aufweist.

## Revendications

1. Cathéter (1) comprenant une tige (2) comportant une lumière (5) communiquant d'une extrémité distale à une extrémité proximale, dans lequel
la tige (2) inclut un élément de renforcement incluant un fil métallique (21) disposé sur au moins une partie entre une surface interne (11) de la tige (2) formant la lumière (5) et une surface externe (31) de la tige (2) et tressé en forme tubulaire,
le corps de renforcement (20) inclut un groupe de fils ronds (24) incluant une pluralité de fils ronds (22), chacun étant un fil métallique (21) présentant une section transversale circulaire, et une pluralité de fils plats (23) croisant le groupe de fils ronds (24), dans lequel les fils ronds et plats font partie du tressage du fil métallique, et
un rapport de surface transversale d'une surface transversale totale de la pluralité de fils ronds (22) du groupe de fils ronds (24) par rapport à une surface transversale totale (B) de la pluralité de fils plats (23) est supérieur à 0,5,
**caractérisé en ce que**
une limite d'élasticité d'un matériau des fils ronds (22) est inférieure à une limite d'élasticité d'un matériau des fils plats (23), et les fils ronds (22) sont déformés au-delà de la limite d'élasticité dans l'état tressé.

2. Cathéter (1) selon la revendication 1, dans lequel le rapport de surface transversale est supérieur à 1.

3. Cathéter (1) comprenant une tige (2) comportant une lumière (5) communiquant d'une extrémité distale à une extrémité proximale, dans lequel
la tige (2) inclut un élément de renforcement incluant un fil métallique (21) disposé sur au moins une partie entre une surface interne (11) de la tige (2) formant la lumière (5) et une surface externe (31) de la tige (2) et tressé en forme tubulaire,
le corps de renforcement (20) inclut un groupe de fils ronds (24) incluant une pluralité de fils ronds (22), chacun étant un fil métallique (21) présentant une section transversale circulaire, et une pluralité de fils plats (23) croisant le groupe de fils ronds (24), dans lequel les fils ronds et plats font partie du tressage du fil métallique, et dans lequel
un rapport d'un diamètre (D) des fils ronds (22) par rapport à une épaisseur (T) des fils plats (23) est supérieur à 1,5,
**caractérisé en ce que** une limite d'élasticité d'un matériau des fils ronds (22) est inférieure à une limite d'élasticité d'un matériau des fils plats (23), et les fils ronds (22) sont déformés au-delà de la limite d'élasticité dans l'état tressé.

4. Cathéter (1) selon l'une quelconque des revendications 1 à 3, dans lequel le nombre de fils ronds (22) est supérieur au nombre de fils plats (23).

5. Cathéter (1) selon l'une quelconque des revendications 1 à 4, dans lequel les fils métalliques (21) qui se croisent ne sont pas reliés entre eux.

6. Cathéter (1) selon l'une quelconque des revendications 1 à 4, dans lequel le fil plat (23) est un fil métallique (21) présentant une section transversale rectangulaire.
